# DEMANDE DE BREVET EUROPEEN

(11) **EP 1 025 831 A1**
(43) Date de publication de la demande: **09.08.2000**
(21) Numéro de dépôt: 00400182.2
(22) Date de dépôt: 24.01.2000
(51) Int. Cl.: A61K 7/02, A61K 7/48

(54) **Composition cosmétique comprenant un poly(hydroxystyrène) et une phase grasse**

(30) Priorité: 05.02.1999 FR 9901384
(71) Demandeur: L'OREAL, 75008 Paris (FR)
(72) Inventeur: Collin, Nathalie, 92330 Sceaux (FR); Yon, Maryline, 75013 Paris (FR)
(74) Mandataire: Kromer, Christophe

(57) **Abrégé**

L'invention a pour objet une composition à application topique comprenant, dans un milieu physiologiquement acceptable, au moins une phase grasse et au moins un poly(hydroxystyrène) éventuellement substitué.

Application au soin et au maquillage de la peau et des fibres kératiniques.

La composition permet d'obtenir un film de très bonne tenue et présentant des propriétés de non transfert. Le film obtenu est notamment résistant aux frottements et au sébum.

## Description

La présente invention a pour objet une composition contenant au moins un poly(hydroxystyrène), destinée en particulier au domaine cosmétique. Plus précisément, l'invention se rapporte à une composition pour le soin ou le maquillage de la peau, y compris les lèvres, ou encore des fibres kératiniques comme les cils, les sourcils et les cheveux, notamment d'êtres humains.

Cette composition peut se présenter sous la forme de mascara, d'eye-liner, de produit pour les lèvres, de fard à joues ou à paupières, de fond de teint, de produit de maquillage du corps, de produit anti-cernes, de composition de protection solaire, de coloration de la peau, de produit de soin de la peau.

Les produits de maquillage ou de soin de la peau ou des lèvres d'êtres humains comme les eye-liners, les fonds de teints, les rouges à lèvres, ou bien encore des cils comme les mascaras, contiennent généralement des corps gras tels que des cires et des huiles, des pigments et/ou charges et, éventuellement des additifs comme des actifs. Ces compositions sont généralement appliquées sur la peau ou les cils sous forme de couche mince conduisant à la formation d'un film.

Le film de maquillage ne présente pas toujours une bonne résistance aux frottements des doigts ou des tissus tels que les vêtements, les serviettes ou les mouchoirs et se désagrège en s'effritant ou en s'étalant. L'effritement du film engendre une perte sensible de l'intensité de la couleur du maquillage, obligeant ainsi la consommatrice à renouveler l'application du produit de maquillage. L'étalement du film forme, quant à lui, une auréole très inesthétique autour de la zone maquillée ; ceci est notamment le cas pour un eye-liner.

D'autre part, ces compositions présentent également l'inconvénient de transférer, c'est-à-dire de se déposer au moins en partie, en laissant des traces sur certains supports avec lesquels elles peuvent être mises en contact, et notamment un verre, une tasse, une cigarette, un vêtement ou la peau. Il s'ensuit une persistance médiocre du film appliqué, nécessitant de renouveler régulièrement l'application de la composition de maquillage. Par ailleurs, l'apparition de traces inacceptables, notamment sur les cols de chemisier, peut écarter certaines femmes de l'utilisation de ce type de maquillage.

De plus, pour les peaux à tendance grasse, la sécrétion de sébum accentue le transfert du film de maquillage sur un support venant en contact avec la peau maquillée. Le film n'est donc pas non plus résistant au sébum. Par exemple, pour un eye-liner, lorsque le bord inférieur de la paupière supérieur vient en contact des autres parties de la paupière, le maquillage transfère sur cette partie de peau, provoquant des salissures inesthétiques et une diminution, voire une disparition, du maquillage, ce qui oblige la consommatrice à renouveler l'application du produit d'eye-liner. Ces désagréments sont encore plus accentués lors de frottements avec les doigts ou des tissus (serviettes, mouchoirs), provoquant une destruction importante, voire totale, du film déjà fragilisé.

En outre, ces compositions ont tendances à migrer dans les rides et ridules de la peau, en particulier autour des yeux et des lèvres, entraînant un effet inesthétique.

Le film de maquillage ne présente plus globalement une bonne tenue dans le temps.

La présente invention a donc pour but de proposer une composition ne présentant pas les inconvénients ci-dessus, et conduisant à la formation d'un film ayant une bonne tenue, résistant aux frottements et/ou au sébum, ne migrant pas et ne transférant pas.

La demanderesse a constaté, de façon tout à fait surprenante, que l'utilisation d'un poly(hydroxystyrène), dans une composition physiologiquement acceptable, pouvait permettre d'obtenir un film de très bonne tenue et présentant de bonnes propriétés de non transfert. Le film obtenu est notamment bien résistant aux frottements, par exemple des doigts ou des tissus, et au sébum. Le film est également souple, flexible, brillant, non collant, ne migre pas et ne transfère pas. En outre, le film présente aussi une bonne résistance à l'eau, lors de baignade ou de douche par exemple, et/ou aux larmes et/ou à la transpiration.

Les poly(para-hydroxystyrène) sont connus dans le document FR-A-2476441 comme agent antifongique et antibactérien. Le document EP-A-605951 décrit également leur utilisation dans une composition de vernis à ongles comprenant un poly(4-hydroxystyrène) pour améliorer l'adhésion sur l'ongle.

De façon plus précise, la présente invention a pour objet une composition à application topique comprenant, dans un support physiologiquement acceptable, au moins une phase grasse et au moins un polymère filmogène, caractérisée par le fait que :
- la phase grasse comprend au moins un corps gras choisi dans le groupe formé par les cires, et les huiles choisies parmi les hydrocarbures ayant au moins 4 atomes de carbone, les composés comportant au moins une liaison C-O et ayant au moins 7 atomes de carbone, les huiles siliconées, les huiles fluorées, et leurs mélanges ,
- le polymère filmogène est un poly(hydroxystyrène) éventuellement substitué, le rapport pondéral d'huile par rapport au poly(hydroxy)styrène étant supérieur ou égal à 0,5 lorsque l'huile est choisie parmi les composés comportant au moins une liaison C-O et ayant au moins 7 atomes de carbone.

Par physiologiquement acceptable, il faut comprendre un milieu compatible avec la peau et/ou les fibres kératiniques, comme un milieu cosmétique.

Un autre objet de l'invention est l'utilisation dans une composition cosmétique, ou pour la fabrication d'une composition à application topique d'au moins un poly(hydroxystyrène) éventuellement substitué pour obtenir un film de bonne tenue et/ou résistant à l'eau et/ou résistant aux frottements et/ou résistant à la transpiration et/ou résistant au sébum et/ou ayant des propriétés de non transfert et/ou ne migrant pas, la composition comprenant au moins une phase grasse telle que définie précédemment.

L'invention a aussi pour objet un procédé cosmétique de maquillage ou de traitement non thérapeutique de la peau et/ou des fibres kératiniques, consistant à appliquer sur ceux-ci une composition telle que décrite précédemment.

L'invention a également pour objet un procédé cosmétique pour limiter le transfert et/ou la migration et/ou pour améliorer la résistance aux frottements et/ou au sébum d'une composition, consistant à introduire dans ladite composition une quantité efficace d'un poly(hydroxystyrène) éventuellement substitué.

Le polymère filmogène utilisé dans la composition selon l'invention peut être un homopolymère ou un copolymère d'au moins un monomère hydroxystyrène éventuellement substitué. Dans la suite de la description, on utilisera le mot poly(hydroxystyrène) pour tout polymère (homo- ou co-polymère) comportant au moins 2 motifs hydroxystyrène.

De préférence, le poly(hydroxystyrène) peut être un poly(para-hydroxystyrène) ou poly(4-hydroxystyrène), et notamment un homopolymère de para-hydroxystyrène (appelé également 4-hydroxystyrène). Dans cet homopolymère, les groupes phényle ne sont pas substitués.

Selon l'invention, au moins un des groupes phényle du poly(hydroxystyrène) peut être substitué par un ou plusieurs radicaux choisis dans le groupe formé par les radicaux alkyles en C₁-C₂₀, alcoxyalkyles en C₁-C₂₀, carboxyalkyles en C₁-C₂₀, phényle, phényle substitué, halogène (notamment chlore, brome, fluor et iode), benzotriazole, nitro, amino.

Par "phényle substitué", on entend un phényle substitué par au moins un substituant choisi dans le groupe formé par les radicaux halogènes (chlore, brome, fluor, iode), amino, nitro, hydroxy, alkyle en C₁-C₂₀, alcoxy (qui signifie un alcoxy linéaire ou ramifié ayant de 1 à 10 atomes de carbone), par exemple, méthoxy, éthoxy, propoxy, isopropoxy, butoxy, isobutoxy, sec-butoxy, tert-butoxy, pentyloxy, isopentyloxy, hexyloxy, heptyloxy, octyloxy, nonyloxy et décyloxy, alkyle halogéné (qui signifie un alkyle linéaire ou ramifié ayant de 1 à 8 atomes de carbone qui est substitué par au moins un halogène), par exemple, chlorométhyle, bromométhyle, fluorométhyle, iodométhyle, chloro-2 éthyle, bromo-2 éthyle, fluoro-2 éthyle, chloro-3 propyle, bromo-3 propyle, fluoro-3 propyle, chloro-4 butyle, fluoro-4 butyle, dichlorométhyle, dibromométhyle, difluorométhyle, diodométhyle, dichloro-2,2 éthyle, dibromo-2,2 éthyle, difluoro-2,2 éthyle, dichloro-3,3 propyle, difluoro-3,3 propyle, dichloro-4,4 butyle, difluoro-4,4 butyle, trichioro méthyle, difluoro-4,4 butyle, trichlorométhyle, trifluorométhyle, trifluoro-2,2,2 éthyle, trifluoro-2,3,3 propyle, tétrafluoro-1,1,2,2 éthyle, et tétrafluoro-2,2,3,3 propyle.

Par radical alkyle, on entend un radical alkyle linéaire ou ramifié en C₁-C₂₀, comme par exemple les radicaux méthyle, éthyle, propyle, isopropyle, butyle, iso-butyle, sec-butyle, tertio-butyle, pentyle, isopentyle, néopentyle, hexyle, heptyle, octyle, éthyl-2 hexyle, 1,1,13,3-tétraméthylbutyle, nonyle, décyle, dodécyle, tétradécyle, nexadécyle, octadécyle et eicosyle.

Selon la présente invention, on entend aussi par poly(hydroxystyrène) des polymères, copolymères et polymères blocs ayant des motifs aromatiques comportant des groupements hydroxy dans leur structure. Ces polymères peuvent comporter d'autres motifs aromatiques ne comportant pas de groupement hydroxy, notamment lorsque le poly(hydroxystyrène) est issu de la copolymérisation de parahydroxystyrène et de styrène.

Les poly(hydroxystyrène) de la composition selon l'invention peuvent être linéaires ou branchés. On peut aussi utiliser un mélange de poly(hydroxystyrène) linéaire et de poly(hydroxystyrène) branché. Ces polymères sont connus et notamment décrits dans les documents US-A-5453483, US-A-5453481, US-A-5554719, US-A-5565544, EP-A-108624. De tels polymères sont vendus sous les dénominations "PHS-E", "PHS-8E01", "PHS-PG", "PHS-N", "PHS-PG-L" par la société CLARIANT.

Le PHS-E, PHS-8E01 et le PHS-PG-L sont un homopolymère linéaire répondant à la formule:

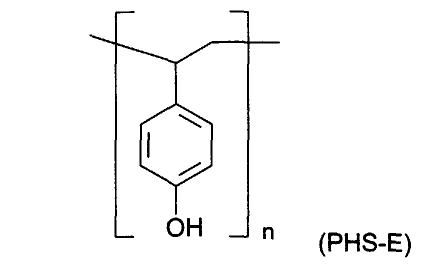

La masse moléculaire du PHS-E est comprise entre 8 000 et 100 000 g/mol.

Le PHS-PG est un polymère répondant à la formule:

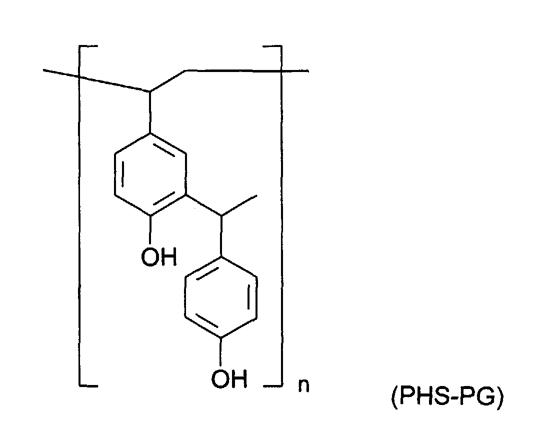

Sa masse moléculaire est comprise entre 4000 et 7 000 g/mol.

Le PHS-N est un polymère branché répondant à la formule:

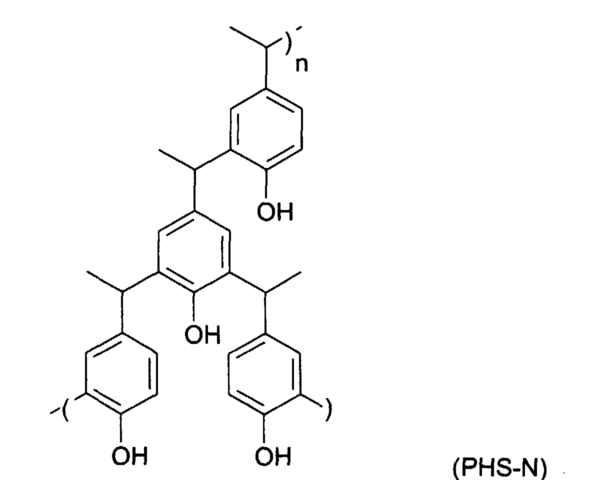

Sa masse moléculaire est comprise entre 4 000 et 7 000 g/mol.

Ledit poly(hydroxystyrène) peut être présent dans la composition en une teneur allant de 0,1 % à 50 % en poids, par rapport au poids total de la composition, de préférence de 0,5 % à 20 % en poids, et mieux de 1 % à 15 % en poids.

La phase grasse de la composition selon l'invention permet d'incorporer facilement ledit copolymère de styrène/acrylate dans la composition.
La phase grasse peut comprendre au moins un corps gras qui peut être liquide, pâteux ou solide à température ambiante (25 °C en général). En particulier, le corps gras peut être choisi parmi les huiles, les cires, les corps gras pâteux, les gommes et leurs mélanges. Le corps gras peut être présent dans la composition en une teneur allant de 1 % à 99,9 % en poids, par rapport au poids total de la composition, de préférence de 10 % à 90 % en poids, et mieux de 20 % à 80 % en poids.

Avantageusement, la phase grasse de la composition comprend au moins un corps gras liquide à température ambiante.

Par " composés comportant au moins une liaison C-O et ayant au moins 7 atomes de carbone", on entend dans la présente demande des composés comportant au moins une liaison simple C-O (liaison de type éther) et/ou une liaison double -C=O (liaison de type carbonyle). Ces composés comprennent notamment les alcools, les aldéhydes, les esters et les acides.

Le corps gras liquide peut être une huile volatile. On entend par "huile volatile" une huile susceptible de s'évaporer à température ambiante d'un support sur lequel elle a été appliquée, autrement dit une huile ayant une tension de vapeur mesurable à température ambiante.

On peut notamment utiliser une ou plusieurs huiles volatiles à température ambiante et pression atmosphérique ayant par exemple une tension de vapeur, a pression et température ambiante > 0 mm de Hg (0 Pa) et en particulier allant de 10⁻³ à 300 mm de Hg (0,13 Pa à 40.000 Pa), à condition que la température d'ébullition soit supérieure à 30°C. Ces huiles volatiles sont favorables à l'obtention d'un film à propriétés "sans transfert" total et de bonne tenue. Ces huiles volatiles facilitent, en outre, l'application de la composition sur la peau, les muqueuses, les phanères. Ces huiles peuvent être des huiles hydrocarbonées, des huiles siliconées, des huiles fluorées, ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles préférées convenant pour la composition selon l'invention sont en particulier les huiles hydrocarbonées ayant de 8 à 16 atomes de carbones, et notamment les isoalcanes en C8-C16 (ou isoparaffines) et les esters ramifiés en C8-C16 comme l'isododécane (encore appelé 2,2,4,4,6-pentaméthylheptane), l'isodécane, l'isohexadécane, le néo pentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées.

Comme huiles volatiles, on peut aussi utiliser les silicones volatiles, comme par exemple les huiles de silicones cycliques et volatiles, notamment celles ayant une viscosité ≤ 8 centistokes (8 10⁻⁶ m²/s), telles que l'octaméthylcyclotétrasiloxane, le décaméthylcyclopentasiloxane, l'hexadécaméthylcyclohexasiloxane, les silicones linéaires volatiles telles que l'octaméthyltrisiloxane, l'heptaméthylhexyltrisiloxane, l'heptaméthyloctyltrisiloxane, le décaméthyltétrasiloxane, ou bien encore les huiles volatiles fluorées telles que le nonafluorométhoxybutane ou le perfluorométhylcyclopentane.

L'huile volatile peut être présente dans la composition selon l'invention en une teneur allant de 0 % à 80 % en poids (notamment de 1 % à 80 %), par rapport au poids total de la composition, de préférence de 0 % à 65 % en poids (notamment de 1 % à 65 %).

Le corps gras liquide peut également être choisi parmi les huiles non volatiles, et notamment les huiles hydrocarbonées et/ou siliconées et/ou fluorées non volatiles.
Comme huile hydrocarbonée non volatile, on peut notamment citer :
- les huiles hydrocarbonées d'origine animale telle que le perhydrosqualène ;
- les huiles hydrocarbonées d'origine végétale telles que les triglycérides liquides d'acides gras de 4 à 10 atomes de carbone comme les triglycérides des acides heptanoïques ou octanoïque, ou encore les huiles de tournesol, de pépins de raisin, de sésame, de maïs, d'abricot, de ricin, d'avocat, d'olive ou de germes de céréales de soja, l'huile d'amande douce, de palme, de colza, de coton, de noisette, de macadamia, de jojoba, les triglycérides des acides caprylique/caprique comme ceux vendus par la société Stéarineries Dubois ou ceux vendus sous les dénominations Miglyol 810, 812 et 818 par la société Dynamit Nobel, l'huile de beurre de karité ;
- les hydrocarbures linéaires ou ramifiés, d'origine minérale ou synthétique tels que la vaseline, les polydécènes, le polyisobutène hydrogéné tel que le parléam ;
- les esters et les éthers de synthèse comme les huiles de formule R₁COOR₂ dans laquelle R₁ représente le reste d'un acide gras supérieur comportant de 6 à 29 atomes de carbone et R₂ représente une chaîne hydrocarbonée contenant de 3 à 30 atomes de carbone, telles que l'huile de Purcellin, le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyl-dodécyle ; les esters de polyols comme le dioctanoate de propylène glycol, le diheptanoate de néopentylglycol, le diisonanoate de diéthylène glycol et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide myristique, l'acide palmitique, l'acide stéarique, l'acide béhénique, l'acide oléique, l'acide linoléique, l'acide linolénique ou l'acide isostéarique ;
et leurs mélanges.

Les huiles de silicone non volatiles utilisables dans la composition selon l'invention peuvent être des huiles de faible viscosité telles que les polysiloxanes linéaires dont le degré de polymérisation est de préférence de 6 à 2000 environ. On peut citer, par exemple, les polydiméthylsiloxanes (PDMS) de viscosité supérieure à 10 mPa.s, les phényl diméthicones, les phényl triméthicones, les polyphénylméthylsiloxanes et leurs mélanges.

Les huiles non volatiles peuvent être présentes dans la composition selon l'invention en une teneur allant de 0 % à 50 % en poids (notamment 0,1 à 50 %), par rapport au poids total de la composition, de préférence de 0 % à 20 % en poids (notamment 0,1 % à 20 %).

Les cires peuvent être choisies parmi les cires d'origine animale, les cires d'origine végétale ou les cires d'origine synthétique.

Les cires susceptibles d'être utilisées dans la composition selon l'invention possèdent en règle générale un point de fusion compris entre 40 et 110 °C et ont une pénétrabilité à l'aiguille allant de 1 à 217. La pénétrabilité à l'aiguille des cires est déterminée selon la norme française NF T 60-123 ou la norme américaine ASTM D 1321, à la température de 25 °C. Selon ces normes, la pénétrabilité à l'aiguille est la mesure de la profondeur, exprimée en dixièmes de millimètre, à laquelle une aiguille normalisée, pesant 2,5 g disposée dans un équipage mobile pesant 97,5 g et placée sur la cire à tester, pendant 5 secondes, pénètre dans la cire.

Parmi les cires d'origine animale, on peut citer les cires d'abeille, les cires de lanoline et les cires d'insectes de Chine.
Parmi les cires d'origine végétale, on peut citer les cires de riz, les cires de Carnauba, de Candellila, d'Ouricurry, les cires de fibres de liège, les cires de canne à sucre, les cires du Japon, la cire de Sumac, la cire de coton.
Parmi les cires d'origine minérale, on peut citer les paraffines, les cires microcristallines, les cires de Montan et les ozokérites.
Parmi les cires d'origine synthétique, on peut utiliser notamment les cires de polyoléfine et notamment les cires de polyéthylène, les cires obtenues par la synthèse de Fischer et Tropsch, les copolymères cireux ainsi que leurs esters, les cires de silicone.
Il est également possible d'utiliser des huiles d'origine animale ou végétale hydrogénées qui répondent toujours aux deux caractéristiques physiques mentionnées précédemment.

Parmi ces huiles, on peut citer les huiles hydrogénées qui sont obtenues par hydrogénation catalytique de corps gras composés de chaîne grasse linéaire ou non en C₈-C₃₂, l'huile de tournesol hydrogénée, l'huile de ricin hydrogénée, l'huile de coprah hydrogénée, l'huile de jojoba hydrogénée, la lanoline hydrogénée et les huiles de palme hydrogénées.
Les cires utilisables selon la présente invention sont de préférence solides et rigides à température inférieure à 50 °C.

La composition selon l'invention peut comprendre de 0 % à 30 % (notamment de 0,1 % à 30 %) en poids de cire, en poids par rapport au poids total de la composition, de préférence de 1 % à 25 % en poids.

De préférence, la composition selon l'invention peut comprendre :
- au moins une cire ayant une pénétrabilité à l'aiguille allant de 1 à 7, 5 (dite cire I), notamment en une teneur allant de 0,1 % à 20 % en poids par rapport au poids total de la composition, et
- au moins une cire ayant une pénétrabilité à l'aiguille supérieure à 7,5 et inférieure ou égale à 217 (dite cire II), notamment en une teneur allant de 0,1 % à 10 % en poids par rapport au poids total de la composition. Ce mélange de cire est notamment approprié lorsque la composition est destinée à être utilisée comme mascara ou eye-liner.

La composition selon l'invention peut comprendre en outre au moins un solvant dudit poly(hydroxystyrène). Ce solvant est avantageusement un solvant polaire et peut être notamment choisi
Le solvant peut être notamment choisi parmi les monoalcools en C₂-C₆, les glycols en C₂-C₈, les monoéthers de diol en C₃-C₆, les monoéthers de dialkylèneglycols en C₄-C₈ (le nombre de carbone indiqué correspondant au nombre total de carbone dans le composé). Comme solvant, on peut citer l'éthanol, l'isopropanol, le propylène glycol, le butylène glycol, l'éthoxyéthanol, le diéthylène glycol. Le solvant peut être présent en une teneur allant de 0 % à 50 % en poids (notamment de 0,1 % à 50 %), par rapport au poids total de la composition.

La composition peut également comprendre au moins une matière colorante comme les composés pulvérulents et/ou les colorants liposolubles, par exemple à raison de 0,01 à 50 % du poids total de la composition. Les composés pulvérulents peuvent être choisis parmi les pigments et/ou les nacres et/ou les charges habituellement utilisés dans les compositions cosmétiques ou dermatologiques.
Avantageusement, les composés pulvérulents représentent de 0,1 à 25 % du poids total de la composition et mieux de 1 à 20 %. Lorsque la composition est sous forme de poudre, elle peut contenir jusqu'à 95 % en poids de composés pulvérulents.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être choisies parmi celles bien connues de l'homme du métier et couramment utilisées dans les compositions cosmétiques.

La composition peut comprendre, en outre, un ou plusieurs actifs notamment cosmétiques ou dermatologiques, tels que les agents hydratants, les vitamines, les acides gras essentiels, les protéines, les céramides, les filtres solaires, les agents anti-radicaux libres, les agents anti-inflamatoires, les agents bronzants.
Bien entendu, l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée. Ces actifs peuvent être par exemple utilisés en une teneur allant de 0,001 % à 20 % en poids, par rapport au poids total de la composition.

La composition selon l'invention peut, de plus, comprendre selon le type d'application envisagée, les constituants classiquement utilisés dans les domaines considérés, qui sont présents en une quantité appropriée à la forme galénique souhaitée.

La composition peut contenir, en outre, tout additif usuellement utilisés dans de telles compositions tels que des épaississants, des parfums, des conservateurs, des tensioactifs, des plastifiants, des polymères filmogènes, différents des poly (hydroxystyrène) définis précédemment, lipophiles ou hydrophiles, solubilisés ou dispersés dans la composition, notamment dispersés dans un milieu aqueux ou un milieu huileux tel que décrite dans les documents EP-A-749747 et EP-A-749746.

Lorsque la phase grasse de la composition selon l'invention comprend au moins un corps gras liquide à température ambiante, la composition peut comprendre, en outre, un agent épaississant de la phase grasse. L'agent épaississant peut être choisi parmi les argiles organomodifiées qui sont des argiles traitées par des composés choisis notamment parmi les amines quaternaires, les amines tertiaires.
Comme argiles organomodifiées, on peut citer les bentonites organomodifiées telles que celles vendues sous la dénomination "Bentone 34" par la société RHEOX, les hectorites organomodifiées telles que celles vendues sous la dénomination "Bentone 27", "Bentone 38" par la société RHEOX.
On peut également employer des silices traitées, des gommes de guar alkylées liposolubles.

Selon un mode de réalisation de l'invention, la composition peut être avantageusement anhydre, et peut contenir moins de 10 % d'eau par rapport au poids total de la composition. Elle peut alors se présenter sous forme de gel huileux, de liquide huileux, de pâte, de produit coulé en stick ou bâton, ou en coupelle, ou bien encore sous forme de poudre.

Selon un autre mode de réalisation, la composition peut comprendre en outre au moins une phase aqueuse et se présenter alors sous forme d'émulsion eau-dans-huile, huile-dans-eau, cire-dans-eau, eau-dans-cire, de dispersion vésiculaire contenant des lipides ioniques et/ou non ioniques. La teneur en eau peut aller de 0 à 95 % (notamment 0,1 % à 95 %) en poids par rapport au poids total de la composition, et mieux de 10 % à 95 % en poids.

Ces compositions à application topique peuvent constituer notamment une composition cosmétique ou dermatologique de protection, de soin ou de traitement de la peau, notamment pour le visage, pour le cou, pour les mains ou pour le corps (par exemple crème de soin, huile solaire, gel corporel), une composition de maquillage, de composition de protection solaire ou une composition de bronzage artificiel.

La composition de maquillage peut être notamment un mascara, un eye-liner, un produit pour les lèvres (rouge à lèvres), un fard à paupières ou à joues, un produit anti-cernes, un fond de teint, un produit de maquillage du corps du type tatouage provisoire ou semi-permanent.

L'invention est illustrée plus en détail dans les exemples suivants.

### Exemple 1 :

On a préparé un mascara ayant la composition suivante :

| | |
|---|---|
| - Cire d'abeille | 3,6 g |
| - Cire de carnauba | 2,9 g |
| - Cire de paraffine | 11,4 g |
| - Poly(para-hydroxystyrène) ( PHS-8E01 de la société CLARIANT) | 2 g |
| - Ethanol | 10 g |
| - Acide stéarique | 5,82 g |
| - Triéthanolamine | 2,4 g |
| - Amino-2 méthyl-2 propane diol-1,3 | 0,5 g |
| - Agents épaississants hydrosolubles | 4,36 g |
| - Polymères filmogène hydrosolubles | 0,35 g |
| - Pigments | 5,45 g |
| - Conservateurs | qs |
| - Eau | qsp 100 g |

Le mascara s'applique facilement sur les cils et permet d'obtenir un maquillage présentant une bonne tenue, résistant aux frottements des doigts et au sébum. En outre, le maquillage ne transfère pas.

### Exemple 2 :

On a préparé un eye-liner ayant la composition suivante :

| | |
|---|---|
| - Cire de Carnauba | 2,3 g |
| - Cire d'abeille | 2,4 g |
| - Cire de polyoléfine (PERFORMA V 260 de PETROLITE) | 3,7 g |
| - Oxyde de fer noir | 11 g |
| - Bentonite | 5,25 g |
| - Carbonate de propylène | 1,78 g |
| - Poly(para-hydroxystyrène) (PHS-8E01 de la société CLARIANT) | 5 g |
| - Copolymère acétate de vinyle/stéarate d'allyle (65/35) (polymère liposoluble) | 7,5 g |
| - Amidon de riz | 1 g |
| - Hydrocarbures paraffiniques et naphténiques légers (Shell Solt de SHELL) | 28,5 g |
| - Hydrocarbures iso-paraffiniques (Isopar E d'ESSO) | qsp 100 g |

On obtient un eye-liner waterproof qui s'applique facilement sur le bord des paupières et laisse, après l'application, un film homogène présentant une bonne tenue dans le temps, résistant à l'eau et à la transpiration. Le film est résistant aux frottements des doigts, ne s'altère pas pendant la journée et ne transfère pas.

### Exemple 3 :

On a préparé un mascara waterproof ayant la composition suivante :

| | |
|---|---|
| - Cire d'abeille | 8,3 g |
| - Cire de carnauba | 4,5 g |
| - Cire de paraffine | 2,2 g |
| - Poly(para-hydroxystyrène) branché ( PHS-N de la société CLARIANT) | 5 g |
| - Ethanol | 10 g |
| - Acide stéarique | 5,82 g |
| - Triéthanolamine | 2,4 g |
| - Amino-2 méthyl-2 propane diol-1,3 | 0,5 g |
| - Bentonite | 5,3 g |
| - carbonate de propylène | 1,74 g |
| - Amidon de riz | 0,84 g |
| - Polylaurate de vinyle (Mexomère PP de CHIMEX) | 0,75 g |
| - Copolymère acétate de vinyle/stéarate d'allyle (65/35) (Mexomère PQ de CHIMEX) | 2,2 g |
| - Dispersion aqueuse de polymère vinylique * | 2,5 g MA |
| - Dispersion aqueuse à 30 % en poids de copolymère acrylate d'éthyle /méthacrylate de méthyle (EUDRAGIT NE 30D de la société ROHM PHARMA) | 2,9 g |
| - Pigments | 4,6 g |
| - Conservateurs | qs |
| - Eau | 4,85 g |
| - Isododécane | qsp 100 g |

| | |
|---|---|
| * Copolymère acétate de vinyle/acide crotonique/tert-butyl-4-benzoate de vinyle (65/10/25) neutralisé à 65 % par l'amino-2 méthyl-2 propanol-1 et plastifié à 25 % par l'adipate de diisopropyle, préparé selon l'enseignement du document EP-A-655234. | |

Le mascara s'applique facilement sur les cils et permet d'obtenir un maquillage présentant une bonne tenue et résistant aux frottements des doigts, au sébum et à l'eau.

## Revendications

1. Composition à application topique comprenant, dans un milieu physiologiquement acceptable, au moins une phase grasse et au moins un polymère filmogène, caractérisée par le fait que :
- la phase grasse comprend au moins un corps gras choisi dans le groupe formé par les cires et les huiles choisies parmi les hydrocarbures ayant au moins 4 atomes de carbone, les composés comportant au moins une liaison C-O et ayant au moins 7 atomes de carbone, les huiles siliconées, les huiles fluorées, et leurs mélanges ,
- le polymère filmogène est un poly(hydroxystyrène) éventuellement substitué, le rapport pondéral d'huile par rapport au poly(hydroxy)styrène étant supérieur ou égal à 0,5 lorsque l'huile est choisie parmi les composés comportant au moins une liaison C-O et ayant au moins 7 atomes de carbone.

2. Composition selon la revendication 1, caractérisée par le fait que le poly(hydroxystyrène) est un poly(para-hydroxystyrène) éventuellement substitué.

3. Composition selon la revendication 1 ou 2, caractérisée par le fait qu'au moins un des groupes phényle du poly(hydroxystyrène) est substitué par un ou plusieurs radicaux choisis dans le groupe formé par les radicaux alkyles en C₁-C₂₀, alcoxyalkyles en C₁-C₂₀, carboxyalkyles en C₁-C₂₀, phényle, phényle substitué, halogène, benzotriazole, nitro, amino.

4. Composition selon l'une quelconque des revendications précédentes, caractérisée par le fait que le poly(hydroxystyrène) est un polymère linéaire ou branché.

5. Composition selon l'une quelconque des revendications précédentes, caractérisée par le fait que le poly(hydroxystyrène) est un mélange de polymère linéaire et de polymère branché.

6. Composition selon l'une quelconque des revendications précédentes, caractérisée par le fait que le poly(hydroxystyrène) a un poids moléculaire moyen en poids allant de 1000 à 500.000, de préférence de 1000 à 100.000, et mieux de 1000 à 30.000.

7. Composition selon l'une quelconque des revendications précédentes, caractérisée par le fait que le poly(hydroxystyrène) est présent en une teneur allant de 0,1 % à 50 % en poids, par rapport au poids total de la composition, de préférence de 0,5 % à 20 % en poids.

8. Composition selon l'une quelconque des revendications précédentes, caractérisée par le fait que le corps gras est présent en une teneur allant de 1 % à 99,9 % en poids, par rapport au poids total de la composition, de préférence de 10 % à 90 % en poids, et mieux de 20 % à 80 % en poids.

9. Composition selon l'une quelconque des revendications précédentes, caractérisée par le fait que la phase grasse comprend au moins une huile volatile.

10. Composition cosmétique selon l'une quelconque des revendications précédentes, caractérisée par le fait que la phase grasse comprend au moins une huile hydrocarbonée volatile.

11. Composition selon l'une quelconque des revendications précédentes, caractérisée par le fait que la phase grasse comprend au moins une huile hydrocarbonée volatile choisie dans le groupe formé par les isoalcanes en C₈-C₁₆ et les esters ramifiés en C₈-C₁₆.

12. Composition selon l'une quelconque des revendications précédentes, caractérisée par le fait que la cire est choisie dans le groupe formé par les cires ayant un point de fusion allant de 40 °C à 110 °C et une pénétrabilité à l'aiguille, à 25 °C, allant de 1 à 217.

13. Composition selon l'une quelconque des revendications précédentes, caractérisée par le fait qu'elle comprend une huile non volatile.

14. Composition selon l'une quelconque des revendications précédentes, caractérisée par le fait qu'elle comprend une huile non volatile choisie dans le groupe formé par le perhydrosqualène, les triglycérides liquides d'acides gras de 4 à 10 atomes de carbone, les hydrocarbures, les esters de formule R₁COOR₂ dans laquelle R₁ représente le reste d'un acide gras supérieur comportant de 6 à 29 atomes de carbone et R₂ représente une chaîne hydrocarbonée contenant de 3 à 30 atomes de carbone, les esters de polyols, les alcools gras liquides à chaîne carbonée ramifiée et/ou insaturée ayant de 12 à 16 atomes de carbone, les acides gras supérieurs, les huiles de silicone non volatiles.

15. Composition selon l'une quelconque des revendications précédentes, caractérisée par le fait qu'elle comprend en outre un solvant du polyhydroxystyrène.

16. Composition selon la revendication 15, caractérisée par le fait que le solvant est choisi dans le groupe formé par les monoalcools en C₂-C₆, les glycols en C₂-C₈, les monoéthers de diol en C₃-C₆, les monoéthers de dialkylèneglycols en C₄-C₈.

17. Composition selon la revendication 15 ou 16, caractérisée par le fait que le solvant est choisi dans le groupe formé par l'éthanol, l'isopropanol, l'éthoxyéthanol, le diéthylène glycol, le propylène glycol, le butylène glycol.

18. Composition selon l'une quelconque des revendications précédentes, caractérisée par le fait qu'elle comprend au moins une matière colorante.

19. Composition selon la revendication 18, caractérisée par le fait que la matière colorante comprend au moins un colorant liposoluble ou un composé pulvérulent choisi dans le groupe formé par les charges, les pigments, les nacres et leurs mélanges.

20. Composition selon l'une quelconque des revendications précédentes, caractérisée par le fait qu'elle comprend, en outre , au moins un additif choisi dans le groupe formé par les épaississants, les parfums, les conservateurs, les actifs, les tensioactifs, les polymères filmogènes lipophiles ou hydrophiles solubilisés ou dispersés dans la composition, différents du poly(hydroxystyrène).

21. Composition selon l'une quelconque des revendications précédentes, caractérisée par le fait qu'elle se présente sous forme de stick ou bâton, de coupelle, d'une pâte, de gel huileux, de liquide huileux, de poudre.

22. Composition cosmétique selon l'une quelconque des revendications précédentes, caractérisée par le fait que la composition est anhydre.

23. Composition selon l'une quelconque des revendications 1 à 21, caractérisée par le fait qu'elle se présente sous forme d'une émulsion eau-dans-huile, huile-dans-eau, de dispersion vésiculaire contenant des lipides ioniques et/ou non ioniques.

24. Composition selon l'une quelconque des revendications précédentes, caractérisée par le fait qu'elle se présente sous la forme d'un produit de soin et/ou de maquillage de la peau et/ou des fibres kératiniques.

25. Composition selon l'une quelconque des revendications précédentes, caractérisée par le fait qu'elle se présente sous la forme d'un mascara, d'un eye-liner, d'un fard à joue ou à paupières, d'un produit pour les lèvres, d'un produit anticernes, d'un maquillage du corps, de produit de protection, de soin ou de traitement de la peau, de composition de protection solaire, de composition de bronzage artificiel.

26. Utilisation dans une composition cosmétique ou pour la fabrication d'une composition à application topique d'au moins un poly(hydroxystyrène) éventuellement substitué, pour obtenir un film de bonne tenue et/ou résistant à l'eau et/ou résistant aux frottements et/ou résistant à la transpiration et/ou résistant au sébum et/ou ayant des propriétés de non transfert, la composition comprenant une phase grasse contenant au moins un corps gras choisi dans le groupe formé par les cires, et les huiles choisies parmi les hydrocarbures ayant au moins 4 atomes de carbone, les composés comportant au moins une liaison C-O et ayant au moins 7 atomes de carbone, les huiles siliconées, les huiles fluorées, et leurs mélanges, le rapport pondéral d'huile par rapport au poly(hydroxy)styrène étant supérieur ou égal à 0,5 lorsque l'huile est choisie parmi les composés comportant au moins une liaison C-O et ayant au moins 7 atomes de carbone.

27. Utilisation selon la revendication 26, caractérisée par le fait que le poly(hydroxystyrène) est un poly(para-hydroxystyrène) éventuellement substitué.

28. Utilisation selon la revendication 27, caractérisée par le fait qu'au moins un des groupes phényle du poly(hydroxystyrène) est substitué par un ou plusieurs radicaux choisis dans le groupe formé par les radicaux alkyles en C₁-C₂₀, alcoxyalkyles en C₁-C₂₀, carboxyalkyles en C₁-C₂₀, phényle, phényle substitué, halogène, benzotriazole, nitro, amino.

29. Utilisation selon l'une quelconque des revendications 26 à 28, caractérisée par le fait que le polyhydroxy styrène est un polymère linéaire ou branché.

30. Procédé cosmétique de maquillage de la peau et/ou des fibres kératiniques, caractérisé par le fait que l'on applique sur la peau et/ou les fibres kératiniques une composition selon l'une quelconque des revendications 1 à 25.

31. Procédé de traitement non thérapeutique de la peau et/ou des fibres kératiniques, caractérisé par le fait que l'on applique sur la peau et/ou les fibres kératiniques une composition selon l'une quelconque des revendications 1 à 25.

32. Procédé cosmétique pour limiter le transfert et/ou la migration et/ou pour améliorer la résistance aux frottements et/ou au sébum d'une composition, consistant à introduire dans ladite composition une quantité efficace d'un poly(hydroxystyrène) éventuellement substitué.
